# EUROPEAN PATENT SPECIFICATION

(11) **EP 2 992 915 B1**
(45) Date of publication and mention of the grant of the patent: **14.12.2022**
(21) Application number: 13881398.5
(22) Date of filing: 01.04.2013
(51) Int. Cl.: A61M 5/00, A61F 5/02, A61F 5/37, A61F 5/01

(54) **MEDICAL AID**
MEDIZINISCHE HILFE
ACCESSOIRE MÉDICAL

(43) Date of publication of application: 09.03.2016
(73) Proprietor: Terumo Kabushiki Kaisha, Tokyo 151-0072 (JP)
(72) Inventor: WADA, Satoshi, Fujinomiya-shi Shizuoka 418-0015 (JP)
(74) Representative: Casalonga
(86) International application number: PCT/JP2013/059921
(87) International publication number: WO 2014/162445

(56) References cited:
- WO-A1-2006/010213
- JP-A- H10 179 662
- JP-U- S53 119 786
- JP-U- S53 119 786
- JP-U- S61 103 146
- JP-Y2- H0 321 227
- US-A- 5 445 603

## Description

### Technical Field

The invention relates to a medical auxiliary tool, and particularly to a medical auxiliary tool for holding a position of a blood vessel when puncturing or stopping of bleeding is performed on a blood vessel, for example, the posterior tibial artery, of the ankle.

### Background Art

When performing therapy, examination, and the like by inserting a medical device, for example, a catheter, into a blood vessel, in general, a puncture needle is punctured through the skin to a blood vessel, and a guide wire is inserted into the blood vessel through the puncture needle which is then removed while leaving the guide wire. Then, an introducer sheath in a state where a dilator is set by being inserted thereinto is inserted into the blood vessel so as to cover around the remaining guide wire which is then removed together with a dilator while leaving the introducer sheath in the blood vessel, and the introducer sheath is indwelled in the blood vessel. Then, a medical device is inserted into the blood vessel through the indwelled introducer sheath. The introducer sheath is removed after therapy, examination and the like is completed, and at this time, stopping of bleeding is performed by pressing the puncture site from the top of the skin.

In such an operation accompanied by the puncturing of a blood vessel, it is necessary to hold the position of the blood vessel even during puncturing of a blood vessel or during stopping of bleeding.

Particularly, when inserting a medical device into a blood vessel, for example, the posterior tibial artery running along the lower part of the malleolus, through the ankle, the position of the posterior tibial artery is not fixed due to relaxed muscles around the posterior tibial artery in a posture in which the joint of the ankle is extended. Therefore, it is desirable to perform puncturing and stopping of bleeding in a state in which the muscles around the posterior tibial artery are extended by bending the joint of the ankle. Here, the state in which the joint of the ankle is bent refers to a state in which the angle formed between a central axis of the lower leg and a plane parallel to the sole of the foot is less than or equal to 90 degrees.

Conventionally, for example, a device which assists lower limb exercise of a patient during or after surgery in order to prevent thrombus by increasing the blood flow of the deep vein is disclosed in PTL 1 as a device which changes the posture of the ankle. This device is configured such that a lower leg fixing portion which fixes the lower leg is disposed on a stand, a sole support plate which supports the sole of the foot is rotatably attached to the stand, and the sole support plate swings relative to the stand through driving force of a motor. WO 2006/010213 discloses a prior art device as defined in the preamble of claim 1.

### Citation List

### Patent Literature

PTL 1: JP-A-2012-29787

### Summary of Invention

### Technical Problem

However, even when insertion of a medical device into the posterior tibial artery is attempted by maintaining the joint of the ankle in a bent state using the device disclosed in PTL 1, it is difficult to perform puncturing and stopping of bleeding on a lower part of the malleolus since a rotation axis for rotating a sole support plate is disposed in the vicinity of the malleolus.

In addition, there is a problem in that the device becomes complicated and the size of the device becomes large due to a mechanism of rotating the sole support plate with respect to a stand, equipment, for example, motor for rotational driving, or the like.

The invention has been made in consideration of such conventional problems, and an object of the invention is to provide a medical auxiliary tool which has a simple structure and with which puncturing or stopping of bleeding can be performed on a blood vessel of the ankle while maintaining the posture of the ankle.

### Solution to Problem

According to the invention, a medical auxiliary tool for holding the position of a blood vessel of the ankle when performing puncturing or stopping of bleeding on the blood vessel, includes: a first fixing portion and a second fixing portion which are respectively fixed to a toe side site and a lower leg side site across the joint of the ankle while exposing the vicinity of the malleolus; and a connecting member which interlocks the first fixing portion and the second fixing portion with each other so as to maintain the joint of the ankle in a bent state.

The medical auxiliary tool may further include: a sock-like member which covers from the toe side site to the lower leg side site and has an opening portion in the vicinity of the malleolus, in which the first fixing portion and the second fixing portion may be integrally formed with the sock-like member.

The first fixing portion and the second fixing portion may be formed of annular members which are respectively fitted to the toe side site and the lower leg side site.

It is preferable that the first fixing portion and the second fixing portion have an anchor portion for locking the connecting member.

One end of the connecting member may be fixed to either of the first fixing portion and the second fixing portion, and the other one of the first fixing portion and the second fixing portion to which the one end of the connecting member has not been fixed may have an anchor portion for locking the other end of the connecting member.

It is preferable that the connecting member has a length adjusting mechanism for adjusting the interlock length between the first fixing portion and the second fixing portion. In this case, the connecting member may be formed of a belt-like member in which both ends of the belt-like member are fixed to each other by being made to overlap each other through the anchor portion of the first fixing portion and the anchor portion of the second fixing portion, and the length adjusting mechanism may be formed of hook-and-loop fasteners which are respectively attached to the both ends of the belt-like member. Similarly, the connecting member may be formed of a belt-like member in which both ends of the belt-like member are fixed to each other by being made to overlap each other through the anchor portion of the first fixing portion and the anchor portion of the second fixing portion, a first hook-and-loop fastener may be attached to one end of one surface of the belt-like member and a second hook-and-loop fastener which is interlocked with the first hook-and-loop fastener is attached across the whole surface of the surface which is different from the surface to which the first hook-and-loop fastener is attached, and the length adjusting mechanism may be formed of the first hook-and-loop fastener and the second hook-and-loop fastener. In the case with such a configuration, since the second hook-and-loop fastener is attached across the whole surface of the surface of the belt-like member which is different from the surface to which the first hook-and-loop fastener is attached, the range in which the first hook-and-loop fastener can be interlocked with the second hook-and-loop fastener becomes wider, and therefore, it is possible to adjust the interlock length between the first fixing portion and the second fixing portion with respect to various users.

In addition, even in a case where the one end of the connecting member is fixed to either of the first fixing portion and the second fixing portion, it is preferable that the connecting member has the length adjusting mechanism for adjusting the interlock length between the first fixing portion and the second fixing portion. In this case, the connecting member may be formed of a belt-like member in which the other end of the connecting member and a part of the connecting member are fixed to each other by being made to overlap each other by passing the other end of the connecting member through the anchor portion, and the length adjusting mechanism may be formed of hook-and-loop fasteners which are attached to at least two places of the belt-like member.

The connecting member may have hooks, which are respectively hooked to the anchor portion of the first fixing portion and the anchor portion of the second fixing portion, at both ends.

In addition, even in a case where the one end of the connecting member is fixed to either of the first fixing portion and the second fixing portion, the connecting member may have a hook, which is hooked to the anchor portion, at the other end of the connecting member.

### Advantageous Effects of Invention

According to the invention, a first fixing portion and a second fixing portion are respectively fixed to a toe side site and a lower leg side site across the joint of the ankle while exposing the vicinity of the malleolus and are interlocked with each other by a connecting member so as to maintain the joint of the ankle in a bent state. Therefore, although the structure of the invention is simple, it is possible to perform puncturing or stopping of bleeding on a blood vessel of the ankle while maintaining the posture of the ankle.

### Brief Description of Drawings

[Fig. 1] Fig. 1 is a side view showing a medical auxiliary tool according to Embodiment 1 of the invention.
[Fig. 2] Fig. 2 is a partial perspective view showing an anchor portion which is used in the medical auxiliary tool according to Embodiment 1. Fig. 2A is a first anchor portion which is disposed in a toe side site and Fig. 2B shows a second anchor portion which is disposed in a lower leg side site.
[Fig. 3] Fig. 3 is a perspective view showing a connecting member which is used in the medical auxiliary tool according to Embodiment 1.
[Fig. 4] Fig. 4 is a side view showing the connecting member which is used in the medical auxiliary tool according to Embodiment 1.
[Fig. 5] Fig. 5 is a perspective view showing a connecting member which is used in a medical auxiliary tool according to a modification example of the Embodiment 1.
[Fig. 6] Fig. 6 is a side view showing a medical auxiliary tool according to another modification example of the Embodiment 1.
[Fig. 7] Fig. 7 is a side view showing a connecting member which is used in a medical auxiliary tool according to Embodiment 2.
[Fig. 8] Fig. 8 is a side view showing a medical auxiliary tool according to Embodiment 3.
[Fig. 9] Fig. 9 is a side view showing a medical auxiliary tool according to Embodiment 4.

### Description of Embodiments

Hereinafter, embodiments of the invention will be described based on the accompanying drawings.

### Embodiment 1

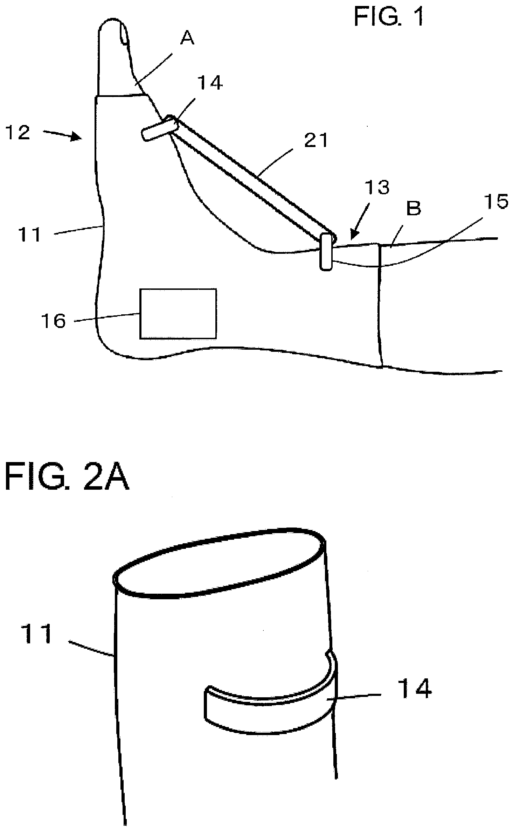

An overall structure of a medical auxiliary tool according to Embodiment 1 is shown in Fig. 1. The medical auxiliary tool includes a sock-like member 11 which covers the ankle from a toe side site A to a lower leg side site B across the joint of the ankle. This sock-like member 11 has a first fixing portion 12 which is mounted on and fixed to the toe side site A and a second fixing portion 13 which is mounted on and fixed to a lower leg side site B. A first anchor portion 14 is attached to the first fixing portion 12 on the instep side of the foot, a second anchor portion 15 is attached to the second fixing portion 13 on the shin side of the foot, and the first fixing portion 12 and the second fixing portion 13 are interlocked with each other by a connecting member 21.

In addition, a rectangular opening portion 16 is formed in the sock-like member 11 in correspondence to the vicinity of the malleolus. The vicinity of the malleolus is exposed by the opening portion 16 through which puncturing and stopping of bleeding are performed on the posterior tibial artery or the like.

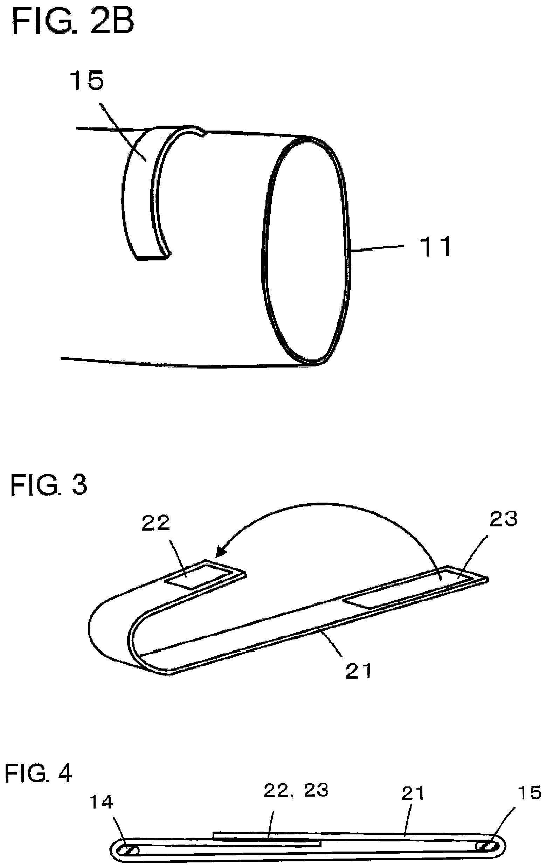

The first anchor portion 14 and the second anchor portion 15 are provided for locking the connecting member 21 and have an arch shape as shown in Figs. 2A and 2B. Both ends of the first anchor portion and the second anchor portion are fixed to the surface of the sock-like member 11 and middle portions thereof are separated from the surface of the sock-like member 11.

As shown in Fig. 3, the connecting member 21 is formed of a flexible belt-like member, and a first hook-and-loop fastener 22 is attached to the surface side of one end of the connecting member 21 and a second hook-and-loop fastener 23 is attached to the rear surface side of the other end. When both ends of the connecting member 21 are made to overlap each other such that the first hook-and-loop fastener 22 which is attached to the surface side of the one end and the second hook-and-loop fastener 23 which is attached to the rear surface side of the other end face each other, by passing this connecting member 21 through the first anchor portion 14 and the second anchor portion 15 as shown in Fig. 4, the first hook-and-loop fastener 22 and the second hook-and-loop fastener 23 are bonded to each other, and therefore, it is possible to interlock the first anchor portion 14 and the second anchor portion 15.

In addition, the first hook-and-loop fastener 22 and the second hook-and-loop fastener 23 which are attached to both ends of the connecting member 21 form a length adjusting mechanism. Therefore, it is possible to adjust the length between the first anchor portion 14 and the second anchor portion 15, that is, the interlock length between the first fixing portion 12 and the second fixing portion 13, by bonding the first hook-and-loop fastener 22 and the second hook-and-loop fastener 23 to each other by mutually shifting the relative positions thereof.

Note that the sock-like member 11 can be formed of cloth materials obtained by interweaving fibers, knitted fabric materials obtained by knitting fibers, or resin materials and rubber materials which have a certain degree of elasticity. It is preferable that the first fixing portion 12 and the second fixing portion 13 are closely contacted to the toe side site A and the lower leg side site B to the extent that it does not become a burden on a patient or a subject. In addition, the opening portion 16 preferably has a size sufficient for carrying out puncturing and stopping of bleeding, and for example, it is possible to set the length along the lower leg to 5 cm to 6 cm and the width to 3 cm to 4 cm.

The first anchor portion 14 and the second anchor portion 15 can be formed of the same material as that of the sock-like member 11, or a resin material or a metallic material which are different from the sock-like member 11.

The connecting member 21 can be formed of various flexible materials, for example, cloth materials, knitted fabric materials, resin materials, and leather materials.

When using the medical auxiliary tool according to Embodiment 1, first, the sock-like member 11 in a state in which the connecting member 21 is not attached thereto is put on the foot of a patient or a subject, the first fixing portion 12 and the second fixing portion 13 are respectively mounted on the toe side site A and the lower leg side site B.

Next, as shown in Fig. 1, the connecting member 21 is passed through the first anchor portion 14 and the second anchor portion 15 of the sock-like member 11 in a state in which the joint of the ankle is bent and the first hook-and-loop fastener 22 and the second hook-and-loop fastener 23 are bonded to each other by overlapping both ends of the connecting member 21 with each other. Accordingly, both ends of the connecting member 21 are fixed to each other by the first hook-and-loop fastener 22 and the second hook-and-loop fastener 23, and the first fixing portion 12 and the second fixing portion 13 are interlocked to each other through the first anchor portion 14 and the second anchor portion 15. As a result, it is impossible to stretch the joint of the ankle of the foot of a patient or a subject and a state in which the joint of the ankle is bent is maintained.

Since the state in which the joint of the ankle is bent is maintained, muscles around the posterior tibial artery are stretched and the position of the posterior tibial artery is held. In this state, puncturing is performed on the posterior tibial artery or the like through the opening portion 16 of the sock-like member 11 which is formed in the vicinity of the malleolus, to perform therapy, examination, and the like. After completing therapy, examination, and the like, it is possible to perform stopping of bleeding through the opening portion 16 of the sock-like member 11.

In this manner, it is possible to efficiently perform the puncturing or stopping of bleeding on a blood vessel of the ankle while maintaining the joint of the ankle in a bent state, by only closely bonding both ends of the connecting member 21 to each other using the first hook-and-loop fastener 22 and the second hook-and-loop fastener 23 by passing the connecting member 21 through the first anchor portion 14 and the second anchor portion 15 of the sock-like member 11 put on the foot.

Note that both ends of the connecting member 21 are closely bonded to each other using the first hook-and-loop fastener 22 and the second hook-and-loop fastener 23, and therefore, it is possible to maintain the joint of the ankle in a bent state which is suitable for an individual patient or subject by absorbing the variation in the shape of the foot depending on a patient or a subject, the variation in the flexibility of the joint of the ankle, and the like.

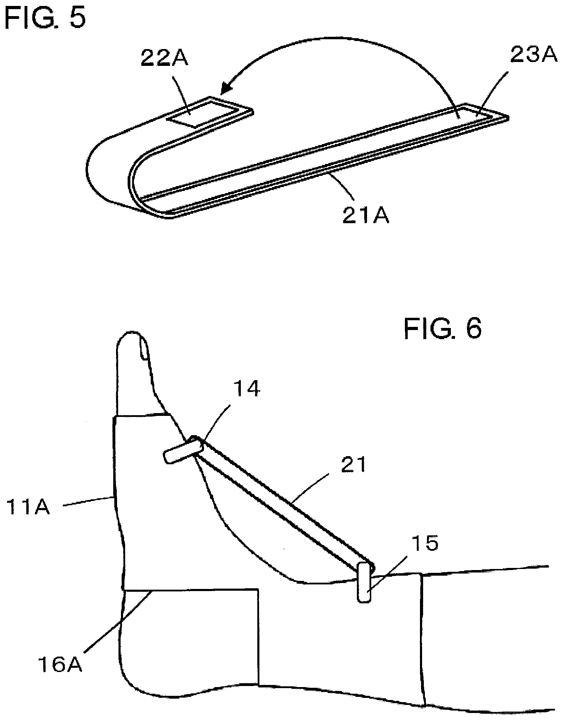

In addition, it is possible to use a connecting member 21A shown in Fig. 5 instead of the connecting member 21. Similarly to the connecting member 21 shown in Fig. 3, the connecting member 21A is formed of a flexible belt-like member, and a first hook-and-loop fastener 22A is attached to one end of one surface of the connecting member 21A and a second hook-and-loop fastener 23A is attached to almost the top of the whole surface over the whole length of the connecting member 21A which is different from the surface to which the first hook-and-loop fastener 22A is attached.

Even in the case with such a configuration, the first hook-and-loop fastener 22A which is attached to the surface side of the one end of the connecting member 21A and the second hook-and-loop fastener 23A which is attached to the rear surface side of the connecting member are bonded to each other by making both ends of the connecting member 21A overlap each other such that the first hook-and-loop fastener 22A and the second hook-and-loop fastener 23A face each other, by passing the connecting member 21A through the first anchor portion 14 and the second anchor portion 15, and therefore, it is possible to interlock the first anchor portion 14 and the second anchor portion 15.

In addition, the first hook-and-loop fastener 22A and the second hook-and-loop fastener 23A which are attached to both of the surfaces of the connecting member 21A form a length adjusting mechanism. Therefore, it is possible to adjust the length between the first anchor portion 14 and the second anchor portion 15, that is, the interlock length between the first fixing portion 12 and the second fixing portion 13, by bonding the first hook-and-loop fastener 22A and the second hook-and-loop fastener 23A to each other by mutually shifting the relative positions thereof. At this time, since the second hook-and-loop fastener 23A is attached to the whole length of the connecting member 21A on the rear surface side, the length adjustable range of the length adjusting mechanism of the connecting member 21A becomes wider and the length of the connecting member 21A can be easily adjusted in accordance with the size, the shape and the like of the body of a user. Therefore, this example is more preferable.

Note that, in the aforesaid Embodiment 1, the rectangular opening portion 16 is formed in the sock-like member 11 in correspondence to the vicinity of the malleolus. However, an opening portion 16A from which the entire heel is exposed as well as the vicinity of the malleolus may be provided as in the case of, for example, the sock-like member 11A shown in Fig. 6.

### Embodiment 2

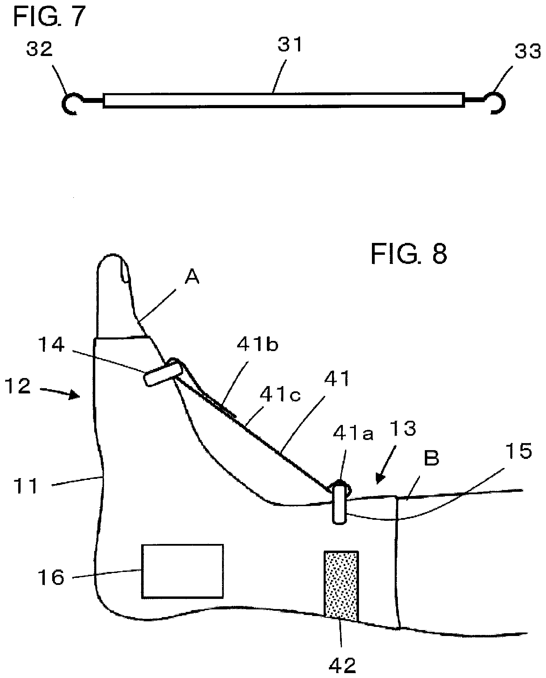

In the above-described Embodiment 1, the connecting member 21 or 21A is passed through the first anchor portion 14 and the second anchor portion 15, and both ends of the connecting member 21 or 21A are bonded to each other by the first hook-and-loop fastener 22 and the second hook-and-loop fastener 23 or by the first hook-and-loop fastener 22A and the second hook-and-loop fastener 23A. However, the invention is not limited thereto. For example, as in the case of a connecting member 31 shown in Fig. 7, hooks 32 and 33, which are respectively hooked to the first anchor portion 14 and the second anchor portion 15, may be provided at both ends of the connecting member.

Both ends of the connecting member 31 are locked with the first anchor portion 14 and the second anchor portion 15 by respectively hooking the hooks 32 and 33 of the connecting member 31 to the first anchor portion 14 and the second anchor portion 15 of the sock-like member 11 which has been put on the foot of a patient or a subject, to maintain the joint of the ankle in a bent state.

In this case, the connecting member 31 may be formed of a flexible material or a rigid body which is not flexible.

Even with such a connecting member 31, it is possible to maintain the state in which the joint of the ankle is bent. However, in a case where the variation in the shape of the foot and the variation in the flexibility of the joint of the ankle depending on an individual patient or subject are large, it is preferable to use the connecting member 21 or 21A having the length adjusting mechanism using the first hook-and-loop fastener 22 and the second hook-and-loop fastener 23 or the first hook-and-loop fastener 22A and the second hook-and-loop fastener 23A as in the above-described Embodiment 1 since it is possible to adjust the interlock length between the first fixing portion 12 and the second fixing portion 13 in accordance with a patient or a subject.

### Embodiment 3

In the aforesaid Embodiment 1 and Embodiment 2, the connecting members 21, 21A, and 31 are members which can be attached to or removed from each of the first anchor portion 14 and the second anchor portion 15. However, the invention is not limited thereto and may have a configuration in which one of both ends of the connecting members is fixed to either of the first anchor portion 14 and the second anchor portion 15.

As shown in Fig. 8, in the connecting member 41 used in Embodiment 3, one end 41a of the connecting member 41 is fixed to the second fixing portion 13 of the sock-like member 11 through the second anchor portion 15 so as not to be removable from the second fixing portion 13, before putting the sock-like member 11 including the first anchor portion 14 and the second anchor portion 15 on the foot of a patient or a subject. Even with such a connecting member 41, the state in which the joint of the ankle is bent is maintained by bonding the other end 41b of the connecting member 41 to an intermediate portion 41c of the connecting member 41 through the first anchor portion 14 using a hook-and-loop fastener.

In this case, the one end 41a of the connecting member 41 may be directly fixed to the second fixing portion 13 of the sock-like member 11 without passing through the second anchor portion 15.

The connecting member 41 may be formed of a flexible material or a rigid body which is not flexible. In addition, it is possible to interlock the other end of the connecting member 41 to the first anchor portion 14 through the hook used in Embodiment 2 instead of bonding the other end 41b of the connecting member 41 which has not been fixed to the sock-like member 11 to the intermediate portion 41c of the connecting member 41 using the hook-and-loop fastener. However, when maintaining the joint of the ankle in a bent state, in a case where the variation in the shape of the foot and the variation in the flexibility of the joint of the ankle depending on an individual patient or subject are large, it is preferable to use the hook-and-loop fastener for interlocking the first anchor portion 14 and the other end 41b of the connecting member 41 which has not been fixed to the sock-like member 11 since it is possible to adjust the interlock length. Here, the intermediate portion 41c of the connecting member 41 refers to a region between the one end 41a of the connecting member 41 and the other end 41b of the connecting member 41. In addition, in the case of Embodiment 3, the connecting member 41 is formed of a belt-like member in which the other end 41b and the intermediate portion 41c of the connecting member 41 are fixed to each other by being made to overlap each other by passing the other end 41b of the connecting member 41 through the first anchor portion 14, and hook-and-loop fasteners are attached to the intermediate portion 41c and the other end 41b on one surface of the belt-like member.

Furthermore, a storage portion 42 for storing the connecting member 41 is formed in the vicinity of the second fixing portion 13 of the sock-like member 11 shown in Fig. 8. The storage portion 42 is formed of, for example, a hook-and-loop fastener, and it is possible to fix the position of the connecting member 41 to the vicinity of the second fixing portion 13 using the hook-and-loop fastener which is attached and bonded to the other end 41b side of the connecting member 41, when the state in which the joint of the ankle is bent is not maintained. According to such a configuration, the connecting member 41 does not disturb a user while walking even if the sock-like member 11 is worn before and after a procedure of performing puncturing or the like.

Note that the invention may be configured such that the storage portion 42 is formed of a bag-like member which is integrally formed with the sock-like member 11, and when the state in which the joint of the ankle is bent is not maintained, the portion on the side of the other end 41b of the connecting member 41 is stored in the storage portion 42 formed of the bag-like member.

In addition, in the aforesaid Embodiment 3, the one end 41a of the connecting member 41 is fixed to the second fixing portion 13 of the sock-like member 11 through the second anchor portion 15 so as not to be removable from the second fixing portion, but the invention is not limited thereto. The one end 41a of the connecting member 41 may be fixed to the first fixing portion 12 of the sock-like member 11 through the first anchor portion 14 so as not to be removable from the first fixing portion, or may be directly fixed to the first fixing portion 12 of the sock-like member 11 without passing through the first anchor portion 14. In this case, it is possible to maintain the joint of the ankle in a bent state, by interlocking the other end 41b of the connecting member 41 with the second anchor portion 15 of the sock-like member 11.

### Embodiment 4

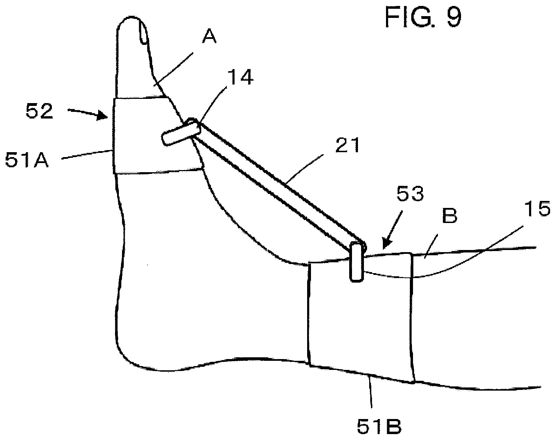

A medical auxiliary tool according to Embodiment 4 is shown in Fig. 9. The medical auxiliary tool uses a first annular member 51A, which is fitted to the toe side site A, and a second annular member 51B, which is fitted to the lower leg side site B, instead of the sock-like member 11 covering the ankle from the toe side site A to the lower leg side site B in the medical auxiliary tool of Embodiment 1 shown in Fig. 1. A first fixing portion 52 which is mounted on the toe side site A is formed by the first annular member 51A and a second fixing portion 53 which is mounted on the lower leg side site B is formed by the second annular member 51B. The first annular member 51A and the second annular member 51B are disposed apart from each other, and the vicinity of the malleolus is greatly exposed between the first annular member 51A and the second annular member 51B.

The first anchor portion 14 and the second anchor portion 15 which are similar to those in Embodiment 1 are respectively attached to the first annular member 51A and the second annular member 51B.

Similarly to the sock-like member 11 of Embodiment 1, the first annular member 51A and the second annular member 51B can be formed of cloth materials obtained by interweaving fibers, knitted fabric materials obtained by knitting fibers, or resin materials and rubber materials which have a certain degree of elasticity. Furthermore, the first annular member 51A and the second annular member 51B can also be formed of flexible belt-like members in which hook-and-loop fasteners are attached to both ends thereof. The belt-like members may be fixed by the hook-and-loop fasteners after winding the belt-like members around the toe side site A and the lower leg side site B, respectively.

Similarly to Embodiments 1 and 2, the state in which the joint of the ankle is bent is maintained even with such a first annular member 51A and second annular member 51B, and therefore, it is possible to efficiently perform puncturing or stopping of bleeding on a blood vessel of the ankle.

### Reference Signs List

11, 11A sock-like member, 12, 52 first fixing portion, 13, 53 second fixing portion, 14 first anchor portion, 15 second anchor portion, 16, 16A opening portion, 21, 21A, 31, 41 connecting member, 22, 22A first hook-and-loop fastener, 23, 23A second hook-and-loop fastener, 32, 33 hook, 41a one end of connecting member, 41b other end of connecting member, 41c intermediate portion of connecting member, 42 storage portion, 51A first annular member, 51B second annular member, A toe side site, B lower leg side site.

## Claims

1. A medical auxiliary tool for holding the position of a blood vessel of the ankle when performing puncturing or stopping of bleeding on the blood vessel, the tool comprising:
a first fixing portion (12, 52) and a second fixing portion (13, 53) which are respectively fixed to a toe side site (A) and a lower leg side site (B) across the joint of the ankle while exposing the vicinity of the malleolus; **characterized in that**
a connecting member (21, 21A, 31, 41) which interlocks the first fixing portion (12, 52) and the second fixing portion (13, 53) with each other so as to maintain the joint of the ankle in a bent state,
the first fixing portion (12, 52) and the second fixing portion (13, 53) are formed of annular members (51A, 51B) which are disposed apart from each other and respectively fitted to the toe side site (A) and the lower leg side site (B) to expose the malleolus between the first annular member (51A) and the second annular member (51B), and
the first fixing portion (12, 52) and the second fixing portion (13, 53) have an anchor portion (14, 15) for locking the connecting member (21, 21A, 31, 41).

2. The medical auxiliary tool according to claim 1, further comprising:
a sock-like member (11, 11A) which covers from the toe side site (A) to the lower leg side site (B) and has an opening portion (16, 16A) in the vicinity of the malleolus,
wherein the first fixing portion (12, 52) and the second fixing portion (13, 53) are integrally formed with the sock-like member (11, 11A).

3. The medical auxiliary tool according to anyone of claims 1 to 2,
wherein one end of the connecting member (21, 21A, 31, 41) is fixed to either of the first fixing portion (12, 52) and the second fixing portion (13, 53), and
wherein the other one of the first fixing portion (12, 52) and the second fixing portion (13, 53) to which the one end of the connecting member (21, 21A, 31, 41) has not been fixed has an anchor portion (14, 15) for locking the other end of the connecting member (21, 21A, 31, 41).

4. The medical auxiliary tool according to anyone of claims 1 to 2,
wherein the connecting member (21, 21A, 31, 41) has a length adjusting mechanism for adjusting the interlock length between the first fixing portion (12, 52) and the second fixing portion (13, 53).

5. The medical auxiliary tool according to claim 4,
wherein the connecting member (21, 21A, 31, 41) is formed of a belt-like member in which both ends of the belt-like member are fixed to each other by being made to overlap each other through the anchor portion (14) of the first fixing portion (12, 52) and the anchor portion (15) of the second fixing portion (13, 53), and
wherein the length adjusting mechanism is formed of hook-and-loop fasteners (22, 22A, 23, 23A) which are respectively attached to the both ends of the belt-like member.

6. The medical auxiliary tool according to claim 4,
wherein the connecting member (21, 21A, 31, 41) is formed of a belt-like member in which both ends of the belt-like member are fixed to each other by being made to overlap each other through the anchor portion (14) of the first fixing portion (12, 52) and the anchor portion (15) of the second fixing portion (13, 53),
wherein a first hook-and-loop fastener (22, 22A) is attached to one end of one surface of the belt-like member and a second hook-and-loop fastener (23, 23A) which is interlocked with the first hook-and-loop fastener (22, 22A) is attached across the whole surface of the surface which is different from the surface to which the first hook-and-loop fastener (22, 22A) is attached, and
wherein the length adjusting mechanism is formed of the first hook-and-loop fastener (22, 22A) and the second hook-and-loop fastener (23, 23A).

7. The medical auxiliary tool according to claim 3,
wherein the connecting member (21, 21A, 31, 41) has a length adjusting mechanism for adjusting the interlock length between the first fixing portion (12, 52) and the second fixing portion (13, 53).

8. The medical auxiliary tool according to claim 7,
wherein the connecting member (21, 21A, 31, 41) is formed of a belt-like member in which the other end of the connecting member (21, 21A, 31, 41) and a part of the connecting member (21, 21A, 31, 41) are fixed to each other by being made to overlap each other by passing the other end of the connecting member (21, 21A, 31, 41) through the anchor portion (14, 15), and
wherein the length adjusting mechanism is formed of hook-and-loop fasteners (22, 22A, 23, 23A) which are attached to at least two places of the belt-like member.

9. The medical auxiliary tool according to anyone of claims 1 to 2,
wherein the connecting member (21, 21A, 31, 41) has hooks (32, 33), which are respectively hooked to the anchor portion (14) of the first fixing portion (12, 52) and the anchor portion (15) of the second fixing portion (13, 53), at both ends.

10. The medical auxiliary tool according to claim 3,
wherein the connecting member (21, 21A, 31, 41) has a hook (32, 33), which is hooked to the anchor portion (14, 15), at the other end of the connecting member (21, 21A, 31, 41).

## Patentansprüche

1. Medizinisches Hilfsmittel zum Halten der Position eines Blutgefäßes des Knöchels bei der Durchführung einer Punktion oder eines Blutstillens an dem Blutgefäß, wobei das Mittel umfasst:
einen ersten Befestigungsabschnitt (12, 52) und einen zweiten Befestigungsabschnitt (13, 53), die jeweils an einer zehenseitigen Stelle (A) und einer unterschenkelseitigen Stelle (B) quer über das Gelenk des Knöchels befestigt sind, wobei die Umgebung des Fußknöchels freigelegt ist;
**dadurch gekennzeichnet, dass**
ein Verbindungselement (21, 21A, 31, 41), das den ersten Befestigungsabschnitt (12, 52) und den zweiten Befestigungsabschnitt (13, 53) miteinander verriegelt, um so das Gelenk des Knöchels in einem gebogenen Zustand zu halten,
der erste Befestigungsabschnitt (12, 52) und der zweite Befestigungsabschnitt (13, 53) aus ringförmigen Elementen (51A, 51B) gebildet sind, die voneinander getrennt angeordnet sind und jeweils an der zehenseitigen Stelle (A) und der unterschenkelseitigen Stelle (B) angebracht sind, um den Fußknöchel zwischen dem ersten ringförmigen Element (51A) und dem zweiten ringförmigen Element (51B) freizulegen, und
der erste Befestigungsabschnitt (12, 52) und der zweite Befestigungsabschnitt (13, 53) einen Verankerungsabschnitt (14, 15) zum Verriegeln des Verbindungselements (21, 21A, 31, 41) aufweisen.

2. Medizinisches Hilfsmittel nach Anspruch 1, ferner umfassend:
ein sockenähnliches Element (11, 11A), das sich von der zehenseitigen Stelle (A) bis zur unterschenkelseitigen Stelle (B) erstreckt und einen Öffnungsabschnitt (16, 16A) in der Nähe des Fußknöchels aufweist,
wobei der erste Befestigungsabschnitt (12, 52) und der zweite Befestigungsabschnitt (13, 53) einstückig mit dem sockenähnlichen Element (11, 11A) ausgebildet sind.

3. Medizinisches Hilfsmittel nach einem der Ansprüche 1 bis 2,
wobei ein Ende des Verbindungselements (21, 21A, 31, 41) an einem von dem ersten Befestigungsabschnitt (12, 52) und dem zweiten Befestigungsabschnitt (13, 53) befestigt ist, und
wobei der andere von dem ersten Befestigungsabschnitt (12, 52) und dem zweiten Befestigungsabschnitt (13, 53), an dem das eine Ende des Verbindungselements (21, 21A, 31, 41) nicht befestigt worden ist, einen Verankerungsabschnitt (14, 15) zum Verriegeln des anderen Endes des Verbindungselements (21, 21A, 31, 41) aufweist.

4. Medizinisches Hilfsmittel nach einem der Ansprüche 1 bis 2,
wobei das Verbindungselement (21, 21A, 31, 41) einen Längeneinstellmechanismus zum Einstellen der Verriegelungslänge zwischen dem ersten Befestigungsabschnitt (12, 52) und dem zweiten Befestigungsabschnitt (13, 53) aufweist.

5. Medizinisches Hilfsmittel nach Anspruch 4,
wobei das Verbindungselement (21, 21A, 31, 41) aus einem bandartigen Element gebildet ist, bei dem beide Enden des bandartigen Elements aneinander befestigt sind, indem sie durch den Verankerungsabschnitt (14) des ersten Befestigungsabschnitts (12, 52) und den Verankerungsabschnitt (15) des zweiten Befestigungsabschnitts (13, 53) einander überlappen, und
wobei der Längeneinstellmechanismus aus Klettverschlüssen (22, 22A, 23, 23A) gebildet ist, die jeweils an den beiden Enden des bandartigen Elements angebracht sind.

6. Medizinisches Hilfsmittel nach Anspruch 4,
wobei das Verbindungselement (21, 21A, 31, 41) aus einem bandartigen Element gebildet ist, bei dem beide Enden des bandartigen Elements aneinander befestigt sind, indem sie durch den Verankerungsabschnitt (14) des ersten Befestigungsabschnitts (12, 52) und den Verankerungsabschnitt (15) des zweiten Befestigungsabschnitts (13, 53) einander überlappen,
wobei ein erster Klettverschluss (22, 22A) an einem Ende einer Oberfläche des bandartigen Elements angebracht ist und ein zweiter Klettverschluss (23, 23A), der mit dem ersten Klettverschluss (22, 22A) verriegelt ist, über die gesamte Oberfläche der Oberfläche angebracht ist, die sich von der Oberfläche unterscheidet, an der der erste Klettverschluss (22, 22A) angebracht ist, und wobei der Längeneinstellmechanismus aus dem ersten Klettverschluss (22, 22A) und dem zweiten Klettverschluss (23, 23A) gebildet ist.

7. Medizinisches Hilfsmittel nach Anspruch 3,
wobei das Verbindungselement (21, 21A, 31, 41) einen Längeneinstellmechanismus zum Einstellen der Verriegelungslänge zwischen dem ersten Befestigungsabschnitt (12, 52) und dem zweiten Befestigungsabschnitt (13, 53) aufweist.

8. Medizinisches Hilfsmittel nach Anspruch 7,
wobei das Verbindungselement (21, 21A, 31, 41) aus einem bandartigen Element gebildet ist, bei dem das andere Ende des Verbindungselements (21, 21A, 31, 41) und ein Teil des Verbindungselements (21, 21A, 31, 41) aneinander befestigt sind, indem sie einander unter Umgehung des anderen Endes des Verbindungselements (21, 21A, 31, 41) durch den Verankerungsabschnitt (14, 15) überlappen, und
wobei der Längeneinstellmechanismus aus Klettverschlüssen (22, 22A, 23, 23A) gebildet ist, die an mindestens zwei Stellen des bandartigen Elements angebracht sind.

9. Medizinisches Hilfsmittel nach einem der Ansprüche 1 bis 2,
wobei das Verbindungselement (21, 21A, 31, 41) Haken (32, 33) an beiden Enden aufweist, die entsprechend in den Verankerungsabschnitt (14) des ersten Befestigungsabschnitts (12, 52) und den Verankerungsabschnitt (15) des zweiten Befestigungsabschnitts (13, 53) eingehakt werden.

10. Medizinisches Hilfsmittel nach Anspruch 3,
wobei das Verbindungselement (21, 21A, 31, 41) einen Haken (32, 33), der an dem Verankerungsabschnitt (14, 15) eingehakt ist, an dem anderen Ende des Verbindungselements (21, 21A, 31, 41) aufweist.

## Revendications

1. Outil auxiliaire médical destiné à maintenir la position d'un vaisseau sanguin de la cheville lors de la réalisation d'une perforation ou d'un arrêt de saignement sur le vaisseau sanguin, l'outil comprenant :
une première partie de fixation (12, 52) et une deuxième partie de fixation (13, 53) qui sont respectivement fixées à un site côté orteils (A) et à un site côté bas de jambe (B) d'un bout à l'autre de l'articulation de la cheville tout en exposant les alentours de la malléole ;
**caractérisé en ce que**
un élément de liaison (21, 21A, 31, 41) qui verrouille la première partie de fixation (12, 52) et la deuxième partie de fixation (13, 53) l'une à l'autre de manière à maintenir l'articulation de la cheville dans un état fléchi,
la première partie de fixation (12, 52) et la deuxième partie de fixation (13, 53) sont formées d'éléments annulaires (51A, 51B) qui sont disposés éloignés l'un de l'autre et respectivement installés sur le site côté orteils (A) et le site côté bas de jambe (B) pour exposer la malléole entre le premier élément annulaire (51A) et
le deuxième élément annulaire (51B), et
la première partie de fixation (12, 52) et la deuxième partie de fixation (13, 53) présentent une partie ancrage (14, 15) pour bloquer l'élément de liaison (21, 21A, 31, 41).

2. Outil auxiliaire médical selon la revendication 1, comprenant en outre :
un élément de type chaussette (11, 11A) qui couvre depuis le site côté orteils (A) jusqu'au site côté bas de jambe (B) et comporte une partie ouverture (16, 16A) aux alentours de la malléole,
dans lequel la première partie de fixation (12, 52) et la deuxième partie de fixation (13, 53) sont formées d'un seul tenant avec l'élément de type chaussette (11, 11A).

3. Outil auxiliaire médical selon l'une quelconque des revendications 1 à 2,
dans lequel une extrémité de l'élément de liaison (21, 21A, 31, 41) est fixée à l'une ou l'autre de la première partie de fixation (12, 52) et de la deuxième partie de fixation (13, 53), et
dans lequel l'autre parmi la première partie de fixation (12, 52) et la deuxième partie de fixation (13, 53) à laquelle n'a pas été fixée la une extrémité de l'élément de liaison (21, 21A, 31, 41) comporte une partie ancrage (14, 15) pour bloquer l'autre extrémité de l'élément de liaison (21, 21A, 31, 41).

4. Outil auxiliaire médical selon l'une quelconque des revendications 1 à 2,
dans lequel l'élément de liaison (21, 21A, 31, 41) présente un mécanisme de réglage de longueur pour régler la longueur de verrouillage entre la première partie de fixation (12, 52) et la deuxième partie de fixation (13, 53.

5. Outil auxiliaire médical selon la revendication 4, dans lequel l'élément de liaison (21, 21A, 31, 41) est formé d'un élément de type ceinture dans lequel les deux extrémités de l'élément de type ceinture sont fixées l'une à l'autre en étant amenées à se chevaucher à travers la partie ancrage (14) de la première partie de fixation (12, 52) et la partie ancrage (15) de la deuxième partie de fixation (13, 53), et
dans lequel le mécanisme de réglage de longueur est formé de systèmes de fermeture autoagrippante (22, 22A, 23, 23A) qui sont respectivement fixés aux deux extrémités de l'élément de type ceinture.

6. Outil auxiliaire médical selon la revendication 4, dans lequel l'élément de liaison (21, 21A, 31, 41) est formé d'un élément de type ceinture dans lequel les deux extrémités de l'élément de type ceinture sont fixées l'une à l'autre en étant amenées à se chevaucher à travers la partie ancrage (14) de la première partie de fixation (12, 52) et la partie d'ancrage (15) de la deuxième partie de fixation (13, 53),
dans lequel un premier système de fermeture autoagrippante (22, 22A) est fixé à une extrémité d'une surface de l'élément de type ceinture et un deuxième système de fermeture autoagrippante (23, 23A) qui est verrouillé avec le premier système de fermeture autoagrippante (22, 22A) est fixé sur toute la surface de la surface qui est différente de la surface à laquelle est fixé le premier système de fermeture autoagrippante (22, 22A), et
dans lequel le mécanisme de réglage de longueur est formé du premier système de fermeture autoagrippante (22, 22A) et du deuxième système de fermeture autoagrippante (23, 23A) .

7. Outil auxiliaire médical selon la revendication 3, dans lequel l'élément de liaison (21, 21A, 31, 41) possède un mécanisme de réglage de longueur pour régler la longueur de verrouillage entre la première partie de fixation (12, 52) et la deuxième partie de fixation (13, 53) .

8. Outil auxiliaire médical selon la revendication 7, dans lequel l'élément de liaison (21, 21A, 31, 41) est formé d'un élément de type ceinture dans lequel l'autre extrémité de l'élément de liaison (21, 21A, 31, 41) et une partie de l'élément de liaison (21, 21A, 31, 41) sont fixées l'une à l'autre en étant amenées à se chevaucher en faisant passer l'autre extrémité de l'élément de liaison (21, 21A, 31, 41) à travers la partie ancrage (14, 15), et
dans lequel le mécanisme de réglage de longueur est formé de systèmes de fermeture autoagrippante (22, 22A, 23, 23A) qui sont fixés à au moins deux endroits de l'élément de type ceinture.

9. Outil auxiliaire médical selon l'une quelconque des revendications 1 à 2,
dans lequel l'élément de liaison (21, 21A, 31, 41) possède des crochets (32, 33), qui sont respectivement accrochés à la partie ancrage (14) de la première partie de fixation (12, 52) et à la partie ancrage (15) de la deuxième partie de fixation (13, 53), au niveau des deux extrémités.

10. Outil auxiliaire médical selon la revendication 3, dans lequel l'élément de liaison (21, 21A, 31, 41) possède un crochet (32, 33), qui est accroché à la partie ancrage (14, 15), au niveau de l'autre extrémité de l'élément de liaison (21, 21A, 31, 41).
